(19) Europäisches Patentamt
European Patent Office
Office européen des brevets

(11) Veröffentlichungsnummer : **0 594 538 A2**

(12) # EUROPÄISCHE PATENTANMELDUNG

(21) Anmeldenummer : **93810716.6**

(22) Anmeldetag : **12.10.93**

(51) Int. Cl.[5] : **C07D 471/06,** G03C 1/73, // (C07D471/06, 249:00, 221:00), (C07D471/06, 235:00, 221:00), (C07D471/06, 221:00, 209:00)

(30) Priorität : **20.10.92 CH 3253/92**

(43) Veröffentlichungstag der Anmeldung : **27.04.94 Patentblatt 94/17**

(84) Benannte Vertragsstaaten : **BE CH DE FR GB IT LI NL SE**

(71) Anmelder : **CIBA-GEIGY AG
Klybeckstrasse 141
CH-4002 Basel (CH)**

(72) Erfinder : **Fischer-Reimann, Evelyn, Dr.
Schutzackerstrasse 64
D-79576 Weil am Rhein (DE)**
Erfinder : **Fischer, Walter, Dr.
Vogesenstrasse 77
CH-4153 Reinach (CH)**

(54) **Photochrome Acridonderivate, Verfahren zu deren Herstellung und deren Verwendung.**

(57) Verbindungen der Formeln I,

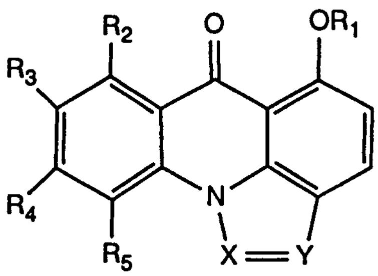

(I),

worin
X und Y je N oder $CR_6$ oder X $CR_6$ und Y N bedeuten ;
$R_1$ unsubstituiertes oder mit $C_1$-$C_{12}$-Alkyl, $C_1$-$C_{12}$-Alkoxy, $C_1$-$C_{12}$-Alkylthio, $C_1$-$C_{12}$-Alkylsulfinyl, $C_1$-$C_{12}$-Alkylsulfonyl, Phenyl, Benzyl, Phenylsulfinyl, Benzylsulfinyl, Phenylsulfonyl, Benzylsulfonyl, -CN, $-CF_3$, Halogen, $-SO_3R_7$, $-CO-R_7$, $-CO_2R_7$, $-CON(R_6)_2$, $-N(R_6)_2$ substituiertes $C_6$-$C_{14}$-Aryl darstellt, wobei das Phenyl und das Phenyl im Benzyl unsubstituiert oder mit $C_1$-$C_4$-Alkyl oder $C_1$-$C_4$-Alkoxy substituiert ist ;
$R_2$, $R_3$, $R_4$ und $R_5$ unabhängig voneinander für H, $-CO_2R_7$ oder Halogen stehen ;
$R_6$ H, unsubstituiertes oder mit $C_1$-$C_4$-Alkyl, $C_1$-$C_4$-Alkoxy, $C_1$-$C_4$-Alkylthio, $-CO_2R_7$ oder Halogen substituiertes $C_1$-$C_{18}$-Alkyl, $C_3$-$C_8$-Cycloalkyl, $C_6$-$C_{14}$-Aryl oder $C_7$-$C_{20}$-Aralkyl ist, oder beide $R_6$ zusammen die Gruppe -CH=CH-CH=CH- bedeuten, wenn X und Y je $CR_6$ sind, wobei diese Gruppe unsubstituiert oder wie für $R_6$ definiert sbstituert ist ;
$R_7$ für H, $C_1$-$C_{18}$-Alkyl, Cyclohexyl, Cyclopentyl, Phenyl, $C_1$-$C_{12}$-Alkylphenyl, Benzyl oder $C_1$-$C_{12}$-Alkylbenzyl steht ; und
die $R_6$ unabhängig voneinander H, $C_1$-$C_{18}$-Alkyl, Cyclohexyl, Cyclopentyl, Phenyl, $C_1$-$C_{12}$-Alkylphenyl, Benzyl, $C_1$-$C_{12}$-Alkylbenzyl ist, oder ein $R_6$ H und das andere $R_6$ die Gruppe $-CO-R_9$ bedeutet, worin $R_9$ unbhängig die Bedeutung von $R_7$ hat, oder beide $R_6$ zusammen Tetramethylen, Pentamethylen, 3-Oxa-1,5-pentylen oder den Rest der Formel $-CH_2CH_2-N(C_1-C_6\text{-Alkyl})CH_2CH_2-$ darstellen.

Die Verbindungen sind irreversibel photochrom und eignen sich als Farbindikatoren, als photoschaltbare Farbfilter oder als Photosensibilisatoren.

EP 0 594 538 A2

Die vorliegende Erfindung betrifft in 4,5-Stellung mit -C=C-, -N=C- oder -N=N-Gruppen überbrückte Acridonderivate, die in 1-Stellung mit einer Aryloxygruppe substituiert sind, ein Verfahren zu deren Herstellung und deren Verwendung als photochrome Systeme zur Kontrastbildung, zur Lichtabsorption und in photoschaltbaren Farbfiltern, sowie als Photosensibilisatoren.

In der EP-A-0 438 376 sind photochrome 5,12-Naphthacendione beschrieben, deren Photochromismus - ein Farbumschlag von gelb nach rot - durch zwei Aryloxygruppen je in den 6,11-Stellungen bedingt ist.

In der EP-A-0 489 689 sind ähnliche photochrome 5,12-Naphthacendione beschrieben, die zusätzlich in den 2-, 3-, 8- und/oder 9-Stellungen mindestens eine weitere Aryloxygruppe oder Halogenatome enthalten.

In der EP-A-0 494 048 sind photochrome Benzothioxanthonoxide beschrieben, die in 11-Stellung und gegebenenfalls in 6-Stellung mit einer Aryloxygruppe substituiert sind.

In der EP-A-0 430 881 sind photochrome 1-Aryloxyacridone beschrieben, deren Farbänderung bei Bestrahlung von farblos nach blau bis violett irreversibel ist. Die Absorptionsmaxima befinden sich in einem relativ langwelligen Bereich. Die thermische Beständigkeit insbesondere der Photoprodukte wird für die Weiterverarbeitung bei Anwendung hoher Temperaturen als zu niedrig empfunden.

Es wurde nun überraschend gefunden, dass in 4,5-Stellung mit einer ungesättigten Gruppe überbrückte Acridone einen irreversiblen Photochromismus und darüberhinaus kurzwelligere Absorptionsmaxima aufweisen. Ferner sind die gebildeten Photoprodukte thermisch wesentlich stabiler.

Ein Gegenstand der Erfindung sind Verbindungen der Formeln I,

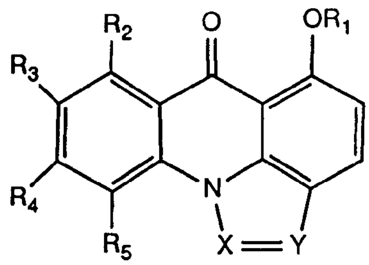

(I),

worin

X und Y je N oder $CR_6$ oder X $CR_6$ und Y N bedeuten;

$R_1$ unsubstituiertes oder mit $C_1$-$C_{12}$-Alkyl, $C_1$-$C_{12}$-Alkoxy, $C_1$-$C_{12}$-Alkylthio, $C_1$-$C_{12}$-Alkylsulfinyl, $C_1$-$C_{12}$-Alkylsulfonyl, Phenyl, Benzyl, Phenylsulfinyl, Benzylsulfinyl, Phenylsulfonyl, Benzylsulfonyl, -CN, -$CF_3$, Halogen, -$SO_3R_7$, -CO-$R_7$, -$CO_2R_7$, -CON$(R_6)_2$, -N$(R_8)_2$ substituiertes $C_6$-$C_{14}$-Aryl darstellt, wobei das Phenyl und das Phenyl im Benzyl unsubstituiert oder mit $C_1$-$C_4$-Alkyl oder $C_1$-$C_4$-Alkoxy substituiert ist;

$R_2$, $R_3$, $R_4$ und $R_5$ unabhängig voneinander für H, -$CO_2R_7$ oder Halogen stehen;

$R_6$ H, unsubstituiertes oder mit $C_1$-$C_4$-Alkyl, $C_1$-$C_4$-Alkoxy, $C_1$-$C_4$-Alkylthio, -$CO_2R_7$ oder Halogen substituiertes $C_1$-$C_{18}$-Alkyl, $C_3$-$C_8$-Cycloalkyl, $C_6$-$C_{14}$-Aryl oder $C_7$-$C_{20}$-Aralkyl ist, oder beide $R_6$ zusammen die Gruppe -CH=CH-CH=CH- bedeuten, wenn X und Y je $CR_6$ sind, wobei diese Gruppe unsubstituiert oder wie für $R_6$ definiert substituiert ist;

$R_7$ für H, $C_1$-$C_{18}$-Alkyl, Cyclohexyl, Cyclopentyl, Phenyl, $C_1$-$C_{12}$-Alkylphenyl, Benzyl oder $C_1$-$C_{12}$-Alkylbenzyl steht; und

die $R_6$ unabhängig voneinander H, $C_1$-$C_{18}$-Alkyl, Cyclohexyl, Cyclopentyl, Phenyl, $C_1$-$C_{12}$-Alkylphenyl, Benzyl, $C_1$-$C_{12}$-Alkylbenzyl ist, oder ein $R_6$ H und das andere $R_8$ die Gruppe -CO-$R_9$ bedeutet, worin $R_9$ unbhängig die Bedeutung von $R_7$ hat, oder beide $R_6$ zusammen Tetramethylen, Pentamethylen, 3-Oxa-1,5-pentylen oder den Rest der Formel -$CH_2CH_2$-N($C_1$-$C_6$-Alkyl)$CH_2CH_2$- darstellen.

$R_2$, $R_3$, $R_4$ und $R_6$ stellen bevorzugt -$CO_2$($C_1$-$C_4$-Alkyl) und besonders bevorzugt H dar.

$R_1$ ist bevorzugt unsubstituiertes oder substituiertes $C_6$-$C_{10}$-Aryl, zum Beispiel Phenyl, 1-oder 2-Naphthyl. Bevorzugt stellt $R_1$ unsubstituiertes oder substituiertes Phenyl dar.

Die Gruppe $R_1$ kann mit einem oder mehreren, bevorzugt 1 bis 3 Resten substituiert sein. Wenn $R_1$ mit Alkyl, Alkoxy, Alkylthio, Alkylsulfinyl oder Alkylsulfonyl substituiert ist, kann das Alkyl linear oder verzweigt sein und bevorzugt 1 bis 6, besonders 1 bis 4 C-Atome enthalten. Beispiele sind Methyl, Ethyl, die Isomeren von Propyl, Butyl, Pentyl, Hexyl, Heptyl, Octyl, Nonyl, Decyl, Undecyl, Dodecyl und die entsprechenden Alkoxy-, Alkylthio-, Alkylsulfinyl- und Alkylsulfonylreste. Bevorzugt sind Methyl, Ethyl, n- und i- Propyl, n-, i- und t-Butyl, Methoxy, Ethoxy, Methylthio, Ethylthio, Methyl- und Ethylsulfinyl, sowie Methyl- und Ethylsulfonyl.

Wenn $R_1$ mit Halogen substituiert ist, handelt es sich bevorzugt um -Br, -Cl und -F.

Falls $R_1$ substituiert ist, sind die Substituenten bevorzugt aus der Gruppe $C_1$-$C_4$-Alkyl, $C_1$-$C_4$-Alkoxy, Phenyl, Benzyl, -$CF_3$, F, Cl, Br, -$SO_3R_7$ und -$CO_2R_7$ ausgewählt, wobei $R_7$ bevorzugt $C_1$-$C_4$-Alkyl bedeutet.

In einer besonders bevorzugten Ausführungsform stellt $R_1$ unsubstituiertes Phenyl dar.

$R_6$ bedeutet als Alkyl bevorzugt $C_1$-$C_{12}$-Alkyl und bevorzugt $C_1$-$C_6$-Alkyl. Das Alkyl kann linear oder verzweigt sein. Bevorzugte Beispiele sind Methyl, Ethyl, n- und i-Isopropyl, n-, i- und t-Butyl, n-Pentyl, 2-Methylbut-4-yl, 3-Methylbut-4-yl, 2,3-Dimethyl-but-4-yl, 1,1-Dimethylprop-3-yl, 1,2-Dimethylprop-3-yl, 2- oder 3-Ethylprop-1-yl, n-Hexyl, 2-Methylpent-1- oder-3- oder -4-yl, 2,3-Dimethylbut-1-yl, 2-Ethylbut-1- oder -4-yl. Bevorzugte Substituenten an $R_6$ als Alkyl sind F, Cl, $C_1$-$C_6$-Alkoxy und -$CO_2$($C_1$-$C_4$-Alkyl), zum Beispiel Methoxy, Ethoxy, n- oder i-Propoxy, n-, i- oder t-Butoxy, -$CO_2CH_3$ und -$CO_2C_2H_5$.

$R_6$ bedeutet als Cycloalkyl bevorzugt Cyclopentyl und Cyclohexyl. Bevorzugte Substituenten für das Cycloalkyl sind $C_1$-$C_4$-Alkyl, $C_1$-$C_4$-Alkoxy, F, Cl und $CO_2$($C_1$-$C_4$-Alkyl), zum Beispiel Methyl, Ethyl, n- oder i-Propyl, n-, i- oder t-Butyl, Methoxy, Ethoxy, n- oder i-Propoxy, n-, i- oder t-Butoxy, -$CO_2CH_3$ und -$CO_2C_2H_5$.

$R_6$ bedeutet als Aryl bevorzugt $C_6$-$C_{10}$-Aryl, zum Beispiel Naphthyl und besonders bevorzugt Phenyl. $R_6$ stellt als Aralkyl bevorzugt $C_7$-$C_{12}$-, besonders bevorzugt $C_7$-$C_9$-Aralkyl und insbesondere Benzyl dar. Bevorzugte Substituenten für das Aryl und das Aryl im Aralkyl sind $C_1$-$C_4$-Alkyl, $C_1$-$C_4$-Alkoxy, F, Cl und -$CO_2$($C_1$-$C_4$-Alkyl), zum Beispiel Methyl, Ethyl, n- oder i-Propyl, n-, i- oder t-Butyl, Methoxy, Ethoxy, n- oder i-Propoxy, n-, i- oder t-Butoxy, -$CO_2CH_3$ und -$CO_2C_2H_5$.

$R_7$ bedeutet als Alkyl bevorzugt $C_1$-$C_{12}$-Alkyl und besonders bevorzugt $C_1$-$C_6$-Alkyl. Das Alkyl kann linear oder verzweigt sein. Bevorzugte Beispiele sind Methyl, Ethyl, n- und i- Isopropyl, n-, i- und t-Butyl, n-Pentyl und n-Hexyl. $R_7$ bedeutet als Alkylphenyl bevorzugt $C_1$-$C_4$-Alkylphenyl und als Alkylbenzyl bevorzugt $C_1$-$C_4$-Alkylbenzyl. Bevorzugte Beispiele sind Methylphenyl, Ethylphenyl, Dimethylphenyl, n- oder i-Propylphenyl, n-, i- oder t-Butylphenyl, Methylbenzyl, Ethylbenzyl, Dimethylbenzyl, n- oder i-Propylbenzyl, n-, i- oder t-Butylbenzyl.

$R_8$ bedeutet als Alkyl bevorzugt $C_1$-$C_{12}$-Alkyl und bevorzugt $C_1$-$C_6$-Alkyl. Das Alkyl kann linear oder verzweigt sein. Bevorzugte Beispiele sind Methyl, Ethyl, n- und i-Isopropyl, n-, i- und t-Butyl, n-Pentyl und n-Hexyl. $R_6$ bedeutet als Alkylphenyl bevorzugt $C_1$-$C_4$-Alkylphenyl und als Alkylbenzyl bevorzugt $C_1$-$C_4$-Alkylbenzyl. Bevorzugte Beispiele sind Methylphenyl, Ethylphenyl, Dimethylphenyl, n- oder i-Propylphenyl, n-, i- oder t-Butylphenyl, Methylbenzyl, Ethylbenzyl, Dimethylbenzyl, n- oder i-Propylbenzyl, n-, i- oder t-Butylbenzyl.

$R_9$ stellt bevorzugt lineares oder verzweigtes $C_1$-$C_6$- Alkyl, Phenyl oder Benzyl dar, zum Beispiel Methyl, Ethyl, n- und i-Isopropyl, n-, i- und t-Butyl, n-Pentyl und n-Hexyl.

Eine bevorzugte Untergruppe sind Verbindungen der Formel I, worin X und Y je N oder $CR_6$ oder X $CR_6$ und Y N bedeuten; $R_1$ unsubstituiertes oder mit $C_1$-$C_6$-Alkyl, $C_1$-$C_6$-Alkoxy, -CN, -$CF_3$, F, Cl, Br, -$SO_3R_7$, -$CO_2R_7$ oder -CON$(R_6)_2$ substituiertes Phenyl darstellt; $R_2$, $R_3$, $R_4$ und $R_6$ unabhängig voneinander für H oder -$CO_2$($C_1$-$C_4$-Alkyl) stehen; $R_6$ H, unsubstituiertes oder mit $C_1$-$C_4$-Alkoxy substituiertes $C_1$-$C_{12}$-Alkyl, unsubstituiertes oder mit $C_1$-$C_4$-Alkyl, $C_1$-$C_4$-Alkoxy, -$CO_2$($C_1$-$C_4$-Alkyl), F, Cl oder Br substituiertes $C_5$- oder $C_6$-Cycloalkyl, Phenyl oder Benzyl ist, oder beide $R_6$ zusammen die Gruppe -CH=CH-CH=CH- bedeuten, wenn X und Y je $CR_6$ sind; $R_7$ H, $C_1$-$C_4$-Alkyl, Cyclohexyl, Phenyl oder Benzyl bedeutet und $R_8$ für $C_1$-$C_4$-Alkyl, Phenyl oder Benzyl steht.

Ein weiterer Gegenstand der Erfindung ist ein Verfahren zur Herstellung von Verbindungen der Formel I, das dadurch gekennzeichnet ist, dass man

a) zur Herstellung von Verbindungen mit X gleich $CR_6$ und Y gleich N

i) eine Verbindung der Formel II

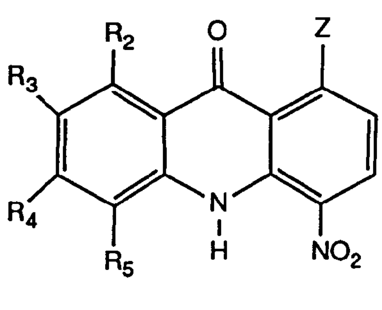

(II)

worin $R_2$, $R_3$, $R_4$ und $R_6$ die zuvor angegebenen Bedeutungen haben und Z für Cl oder Br steht, in Gegenwart eines polaren aprotischen oder protischen Lösungsmittels und bei erhöhter Temperatur mit einer Verbindung der Formel $R_1O^{\ominus}M^{\oplus}$ umsetzt, worin $R_1$ die zuvor angegebenen Bedeutungen hat und $M^{\oplus}$ für ein Alkalimetall- oder ein Ammoniumkation steht,

ii) die gebildete Verbindung der Formel III

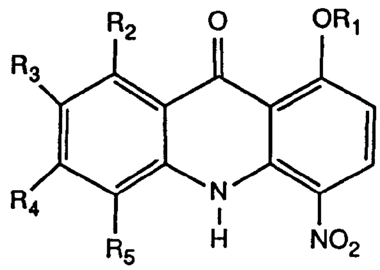

(III)

zu einer Verbindung der Formel IV reduziert

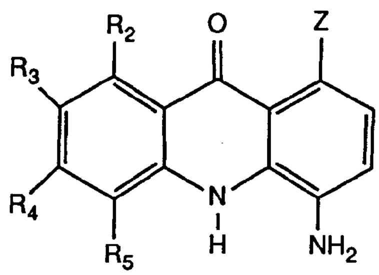

(IV),

und

iii) die Verbindung der Formel IV danach mit einer Carbonsäure der Formel $R_6$-$CO_2H$ umsetzt;

b) zur Herstellung von Verbindungen der Formel I, worin X und Y je N oder $CR_6$ bedeuten, eine Verbindung der Formel V

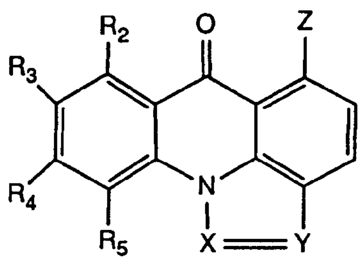

(V),

worin $R_2$, $R_3$, $R_4$, $R_6$ und Z die zuvor angegebenen Bedeutungen haben, in Gegenwart eines polaren aprotischen oder protischen Lösungsmittels und bei erhöhter Temperatur mit einer Verbindung der Formel $R_1O^{\ominus}M^{\oplus}$ umsetzt, worin $R_1$ die zuvor angegebenen Bedeutungen hat und $M^{\oplus}$ für ein Alkalimetall- oder ein Ammoniumkation steht.

Die Verbindungen der Formel II und der Formel V, worin X und Y je N bedeuten, sind von J. Romanowski et al. im Polish Journal of Chemistry 54, Seiten 605 bis 610 beschrieben.

Die Herstellung von Verbindungen der Formel V, worin X und Y je $CR_6$ bedeuten, kann zum Beispiel erfolgen, in dem man

i) eine Verbindung der Formel A

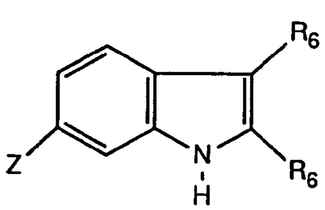

(A)

worin $R_6$ und Z die zuvor angegebenen Bedeutungen haben, mit Orthoiodbenzoesäure der Formel B

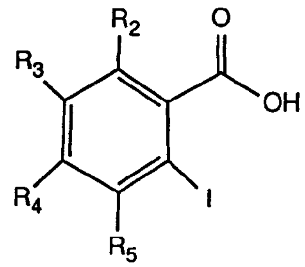

(B)

worin $R_2$, $R_3$, $R_4$ und $R_5$ die zuvor angegebenen Bedeutungen haben, zu einer Verbindung der Formel C

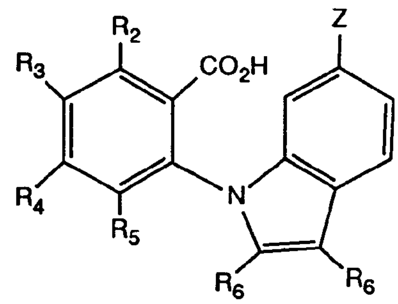

(C)

umsetzt, und

ii) die Verbindung der Formel C durch intramolekulare Friedel-Crafts-Reaktion zu einer Verbindung der Formel D

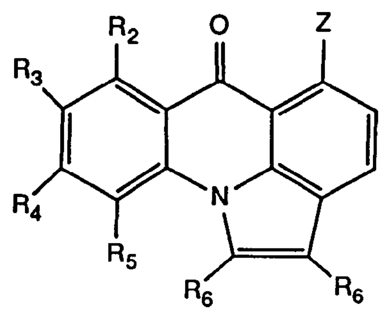

(D)

kondensiert. Das Verfahren ist von A. Echot et al. im J. Prakt. Chem. II, 104, Seite 8 (1992) beschrieben.

Die Umsetzung der Stufe i) im Verfahren a) beziehungsweise im Verfahren b) ist an sich bekannt. Beispiele für $M^{\oplus}$ sind $Li^{\oplus}$, $Na^{\oplus}$, $K^{\oplus}$ und tertiäres Ammonium, zum Beispiel Triethyl-, Trimethyl-, Tri-n-propyl- oder Tri-n-butylammonium.

Die Verfahrenstufe i) wird bevorzugt bei Temperaturen von 50 bis 200°C, besonders 50 bis 150°C durchgeführt. Die Salze der Formel $R_9O^{\ominus}M^{\oplus}$ können als solche eingesetzt werden oder durch Umsetzung eines entsprechenden Phenols mit einer Alkalimetallbase oder Alkalimetallcarbonaten oder Aminen in situ im Reaktionsgemisch erzeugt werden. Die Salze können in äquimolaren Mengen oder im Ueberschuss, z.B. bis zu 40 Mol-% Ueberschuss eingesetzt werden.

Geeignete Lösungsmittel sind z.B. N-substituierte Carbonsäureamide und Lactame (z.B. Dimethylformamid oder N-Methylpyrrolidon), Sulfoxide und Sulfone (z.B. Dimethylsulfoxid, Tetramethylensulfon) oder Ether (z.B. n-Dipropylether, n-Dibutylether, Tetrahydrofuran oder Dioxan). Die Reaktion kann auch in einem Überschuss eines Phenols $R_1OH$ durchgeführt werden, das dann gleichzeitig als Lösungsmittel dient.

Die Reduktion in der Verfahrensstufe ii) kann in an sich bekannter Weise katalytisch mit Raney-Nickel oder Edelmetallkatalysatoren, mit Hydrazin, mit unedlen Metallen oder Metallen niedriger Oxidationsstufen erfolgen, wobei im allgemeinen je nach Reduktionsmittel Temperaturen von Raumtemperatur bis etwa 200 °C, bevorzugt 150 °C eingehalten werden. Die Reaktion wird zweckmässig in einem Lösungsmittel durchgeführt.

Die Umsetzung in der Verfahrensstufe iii) mit einer Carbonsäure $R_6CO_2H$ ist an sich bekannt und von W. M. Cholody et al. im J. Med. Chem 33, Seite 49 (1990) beschrieben. Die Reaktion kann in Gegenwart eines

inerten Lösungsmittels und bei Temperaturen von 40 bis 200 °C durchgeführt werden.

Verbindungen der Formel I, worin X und Y je für N stehen, können auch durch die an sich bekannte Diazotierung von Verbindungen der Formel IV in zum Beispiel wässrig-saurer Lösung mit $NaNO_2$ erhalten werden, wie es von W. M. Cholody et al. im J. Med. Chem. 33, Seite 2852 (1990) beschrieben ist.

Die Verbindungen der Formel I sind farblos und kristallin sowie gut löslich in dipolar aprotischen Lösungsmitteln und Polymeren. Bei der Bestrahlung mit Licht einer Wellenlänge von etwa 300 bis 400 nm wird ein irreversibler Farbumschlag nach gelb (X und Y in Formel I sind je N, $\lambda_{max}$= ca. 440 nm), rot (X und Y in Formel I sind $CR_6$ und N, $\lambda_{max}$= ca. 530 nm) oder blau (X und Y in Formel I sind je $CR_6$, $\lambda_{max}$= ca. 600 nm) beobachtet.

Bei der Bestrahlung bilden sich die Umlagerungsprodukte der Formel

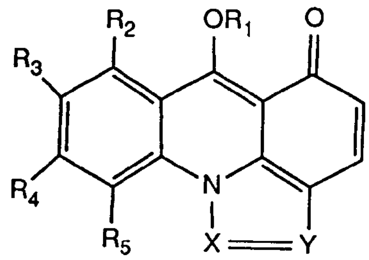,

die eine überraschend hohe Thermostabilität aufweisen. Die Umwandlungsgeschwindigkeit ist überraschend hoch und kann je nach Menge, Dicke der Probe und Strahlungsintensität unter 3 Sekunden liegen. Die Verbindungen der Formel I sind wirksame Photoinitiatoren und Photosensibilisatoren für photopolymerisierbare Systeme, die ethylenisch ungesättigte Doppelbindungen enthalten.

Ein weiterer Gegenstand der Erfindung ist die Verwendung von Verbindungen der Formeln I als photochrome Systeme zur Kontrastbildung oder Lichtabsorption.

Die Verbindungen der Formel I können als Photoinitiatoren und besonders Photosensibilisatoren in photopolymerisierbaren Systemen verwendet werden, wobei sie gleichzeitig als Farbindikatoren wirken. So ist es möglich, belichtete Produkte (z.B. Schutzschichten, Druckplatten, Offsetdruckplatten, gedruckte Schaltungen, Lötstoppmasken) zu markieren und von unbelichteten Produkten zu unterscheiden, und ferner bei einer Produktekontrolle fehlerhaft belichtete Produkte vor oder nach der Entwicklung auszusondern.

Der erhebliche Vorteil bei der Verwendung als Farbindikatoren liegt in der Erhöhung der Sensibilisatorwirkung. Ueblicherweise als Farbumschlagsysteme eingesetzte Zusätze bewirken im allgemeinen eine Erniedrigung der Photosensibilität.

Die Verbindungen der Formeln I können auch als solche, in Lösung oder in Polymeren eingearbeitet, als Photofarbindikatoren oder als Photoschaltelemente verwendet werden.

Die Verbindungen der Formel I können auch in organischen oder anorganischen Gläsern als photoschaltbare Farbfilter verwendet werden, z.B. in Gläsern für Sonnenbrillen, Kontaktlinsen, Fenstern und Spiegeln.

Ein weiterer Gegenstand der Erfindung ist eine strahlungsempfindliche Zusammensetzung, enthaltend

a) ein strahlungsempfindliches organisches Material, und

b) mindestens eine Verbindung der Formeln I.

Die Verbindungen der Formel I können in einer Menge von 0,001 bis 20 Gew.-%, besonders 0,001 bis 10 Gew.-% und insbesondere 0,01 bis 5 Gew.-% enthalten sein, bezogen auf die Komponente a).

Strahlungsempfindliche und damit auch photostrukturierbare Materialien sind bekannt. Es kann sich um Positiv- oder Negativsysteme handeln. Solche Materialien sind z.B. von G.

E. Green et al. in J. Macromol. Sci.; Revs. Macromol. und Chem., C21(2), 187-273 (1981 bis 1982) und von G.A. Delzenne in Adv. Photochem., 11, S. 1-103 (1979) beschrieben worden.

Vorzugsweise handelt es sich bei dem strahlungempfindlichen organischen Material um a1) eine nichtflüchtige monomere, oligomere oder polymere Substanz mit photopolymerisierbaren oder photodimerisierbaren ethylenisch ungesättigten Gruppen, a2) um ein kationisch härtbares System oder a3) um photovernetzbare Polyimide.

Photopolymerisierbare Substanzen sind z.B. Acryl- und besonders Methacrylsäureester von Polyolen, z.B. Ethylenglykol, Propandiol, Butandiol, Hexandiol, Di(hydroxymethyl)-cyclohexan, Polyoxyalkylendiole wie z.B. Di-, Tri- oder Tetraethylenglykol, Di- oder Tri-1,2-propylenglykol, Trimethylolmethan, -ethan oder -propan und Pentaerythrit, die alleine, in Mischungen und in Abmischung mit Bindemitteln verwendet werden können.

Photodimerisierbare Substanzen sind z.B. Homo- und Copolymere, die Zimtsäuregruppen oder substituierte Maleinimidylverbindungen in Seitengruppen oder Chalkongruppen in der Polymerkette enthalten.

Bevorzugt sind solche Zusammensetzungen, worin Komponente a1) ein Homo- oder Copolymer von

Acryl-, Methacryl- oder Maleinsäureestern ist, deren Estergruppen einen Rest der Formel

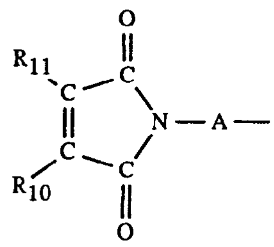

enthalten, worin A für unsubstituiertes oder mit Hydroxyl substituiertes lineares oder verzweigtes $C_2$-$C_{12}$-Alkylen, Cyclohexylen oder Phenylen steht, und $R_{10}$ und $R_{11}$ unabhängig voneinander Cl, Br, Phenyl oder $C_1$-$C_4$-Alkyl bedeuten, oder $R_{10}$ und $R_{11}$ zusammen Trimethylen, Tetramethylen oder

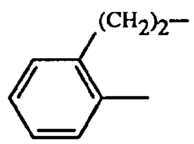

bedeuten. Solche Polymere sind z.B. in der US-A-4 193 927 beschrieben.

Die photopolymerisierbaren oder photodimerisierbaren Substanzen können weitere für die Verarbeitung oder Anwendung übliche Additive enthalten, sowie zusätzlich andere Photoinitiatoren oder Photosensibilisatoren.

Bei den kationisch härtbaren Systemen handelt es sich bevorzugt um Epoxidverbindungen mit mindestens 2 Epoxidgruppen im Molekül, denen ein Photoinitiator einverleibt ist. Geeignete Photoinitiatoren sind z.B. Cyclopentadienyl-Aren-Metallsalze, Cyclopentadienylmetallcarbnoyl-salze und Oniumsalze, die z.B. in den zuvor erwähnten Publikationen beschrieben sind. Die härtbaren Systeme können für die Verarbeitung und Anwendung übliche Zusatzstoffe enthalten.

Photoempfindliche Polyimide sind z.B. in der DE-A-1 962 588, EP-A-0 132 221, EP-A- 0 134 752, EP-A-0 162 017 und EP-A-0 182 745 beschrieben.

Die erfindungsgemässe Zusammensetzung wird nach bekannten Methoden als Schicht auf Substrate aufgebracht und entweder durch flächige Bestrahlung eine Schutzschicht oder durch Bestrahlung unter einer Photomaske oder durch ortsaufgelöste Bestrahlung mittels eines geführten Laserstrahls oder durch holographische Verfahren und nachfolgende Entwicklung ein Reliefbild erzeugt.

Ein weiterer Gegenstand der Erfindung ist eine Zusammensetzung, enthaltend

a) ein farbloses organisches Lösungsmittel, ein Polymer oder ein organisches Glas oder ein Verbundglas, und

b) gelöst, eingemischt oder als Schicht auf mindestens einer Oberfläche eine Verbindung der Formel I. Die Komponente b) ist bevorzugt in einer Menge von 0,001 bis 20 Gew.-%, besonders 0,001 bis 10 Gew.-% und insbesondere 0,01 bis 5 Gew.-% enthalten, bezogen auf Komponente a). Organische Lösungen können zur Beschichtung von anderen Substanzen verwendet werden, z.B. festen Substraten wie zum Beispiel anorganischen Gläsern, die dann als photoschaltbare Substrate verwendet werden können. Die Verbindungen der Formel I können auch auf Substrate aufsublimiert werden. Die beschichteten Substrate können mit einer Schutzschicht aus z.B. transparenten Polymeren versehen werden. Feste Substrate können auch mit Zusammensetzungen beschichtet werden, die ein Polymer und mindestens eine Verbindung der Formel I enthalten. Geeignete Lösungsmittel sind z.B. Kohlenwasserstoffe, halogenierte Kohlenwasserstoffe, Ketone, Carbonsäureester und Lactone, N-alkylierte Säureamide und Lactame, Alkanole und Ether.

Geeignete Polymere sind z.B. Duroplaste, Thermoplaste und strukturell vernetzte Polymere. Die Polymeren sind bevorzugt transparent. Solche Polymere und organische Gläser sind dem Fachmann geläufig. Die Einarbeitung der erfindungsgemässen Verbindungen erfolgt nach üblichen Methoden, z.B. mit Lösungsverfahren und Entfernen des Lösungsmittels, Kalandrieren oder Extrusion. Die erfindungsgemässen Verbindungen können den Substraten auch vor, während oder nach deren Herstellung einverleibt werden.

Ein weiterer Gegenstand der Erfindung ist ein Verfahren zur Herstellung von gefarbten Materialien unter Einwirkung von Licht, dadurch gekennzeichnet, dass man dem Material eine Verbindung der Formel I einverleibt und darauf das Material mit Licht bestrahlt.

Ferner ist ein Gegenstand der Erfindung die Verwendung von Verbindungen der Formel I als Photosensibilisatoren und Farbindikatoren oder photoschaltbare Farbfilter bei Lichteinwirkung.

Die nachfolgenden Beispiele erläutern die Erfindung.

A) Herstellungsbeispiele

Beispiel A1: Herstellung von 5-Phenoxy-6H-v-triazolo[4.5,1-de]acridin-6-on.

a) 4-Nitro-1-(4-phenoxy)acridon

10 g (36 mmol) 1-Chlor-4-nitroacridon, 7,2 g (43 mmol) Phenol und 11 g (79 mmol) wasserfreies $K_2CO_3$ werden in 60 ml Dimethylformamid 2 Stunden auf 80 °C erhitzt. Die abgekühlte Reaktionsmischung wird in Wasser gegossen und der gebildete Niederschlag mit $CH_2Cl_2$ extrahiert. Die organische Phase wird mit wässriger $NaHCO_3$ und dann mit gesättigter Kochsalzlösung gewaschen, danach mit $Na_2SO_4$ getrocknet und eingedampft. Der Rückstand wird in $CH_2Cl_2$ gelöst und mit Hexan ausgefallt. Man erhält (93%) Produkt mit einem Schmelzpunkt von 236-238 °C.

b) 4-Amino-1-phenoxyacridon

10 g (30 mmol) 4-Nitro-1-(4-phenoxy)acridon werden in 400 ml Tetrahydrofuran gelöst und unter Zusatz von 4 g Raney-Nickel bei Raumtemperatur und Normaldruck hydriert. Die erhaltene Suspension wird eingeengt und der Rückstand mit Terahydrofuran extrahiert. Nach dem Verdampfen des Lösungsmittels verbleiben 5,9 g (65%) Rohprodukt, das so weiterverwendet wird.

c) 5-Phenoxy-6H-v-triazolo[4,5,1-de]acridin-6-on

Eine Suspension von 2 g 4-Amino-1-phenoxyacridon in 22 ml konzentrierter Salzsäure wird 30 min bei Raumtemperatur gerührt. Dann gibt man innerhalb von 30 min eine Lösung von 0,6 g (8,8 mmol) $NaNO_2$ in 10 ml Wasser zu und rührt 4 Stunden bei Raumtemperatur. Der gebildete Niederschlag wird abfiltriert und aus Dimethylformamid/Wasser umkristallisiert. Man erhält 1 g (82%) der Titelverbindung, Schmelzpunkt 190-195 °C.

Beispiel A2: Herstellung von 5-Phenoxyimidazo[4.5.1-de]acridin-6-on.

2g (6,6 mmol) 4-Amino-1-phenoxyacridon werden in 22 ml Ameisensäure 4 Stunden am Rückfluss erhitzt. Nach dem Abkühlen wird das Reaktionsgemisch mit 250 ml Wasser verdünnt und mit Natronlauge auf pH 10 gestellt. Man extrahiert mit Toluol, wäscht die organische Phase mit konzentrierter Kochsalzlösung, trocknet mit $Na_2SO_4$ und dampft ein. Man erhält 1,7 g (82%) der Titelverbindung, Schmelzpunkt 233-235 °C.

Beispiel A3: Herstellung von 1-(p-Methoxybenzyl)-5-phenoxyimidazo- [4,5,1-de]acridin-6-on.

Es wird wie in Beispiel A2 verfahren und p-Methoxyphenylessigsäure verwendet. Ausbeute: 31 %, Schmelzpunkt 155-160 °C.

Beispiel A4: Herstellung von 1-(Ethoxymethyl)-5-phenoxyimidazo[4,5,1-de]acridin-6-on.

Es wird wie in Beispiel A2 verfahren und Methoxyessigsäure verwendet. Ausbeute: 9%, Schmelzpunkt 145-149 °C.

Beispiel A5: Herstellung von 5-(p-Ethoxycarbonylphenoxy)imidazo[4,5,1-de]acridin-6-on.

Es wird wie in Beispiel A2 verfahren und 4-Amino-1-(p-ethoxycarbonylphenoxy)acridon verwendet. Ausbeute: 86%, Schmelzpunkt 190-193 °C.

Beispiel A6: Herstellung von 1-iso-Butyl-5-phenoxyimidazo[4,5,1-de]acridin-6-on.

4 g (13,2 mmol) 4-Amino-1-phenoxyacridon werden mit 60 ml Isovaleriansäure in 30 ml Brombenzol 60 h am Rückfluss erhitzt. Das abgekühlte Reaktionsgemisch wird in wässrige NaOH (30%) gegossen, der Niederschlag abfiltriert, mit Wasser gewaschen und getrocknet. Das Produkt wird aus Toluol umkristallisiert. Ausbeute 1,3 g (27%), Schmelzpunkt 203-206 °C.

Beispiel A7: Herstellung von 1-(p-Methoxyphenyl)-5-phenoxy-imidazo[4,5,1-de]acridin-6-on.

Es wird gemäss Beispiel A6 verfahren und p-Methoxybenzoesäure verwendet. Ausbeute 1,5 %, Schmelzpunkt 218-222 °C.

Beispiel A8: Herstellung von 1-(p-Methoxycarbonylphenyl)-5-phenoxy-imidazo[4,5,1-de]acridin-6-on.

Es wird gemäss Beispiel A6 verfahren und p-Methoxycarbonylbenzoesäure verwendet. Ausbeute 3%, Schmelzpunkt 236-239 °C.

Beispiel A9: Herstellung von 1-Phenoxy-4,10-o-phenylenacridon.

a) N-(2-Carboxyphenyl)-2-chlorcarbazol.

Eine Mischung aus 1,2 g (6 mmol) 2-Chlorcarbazol, 1,48 g (6 mmol) o-Iodbenzoesäure, 1,64 g (7,2 mmol) $K_2CO_3$ und 95 mg (1,2 mmol) CuO in 10 ml Nitrobenzol wird 5 h bei 220 °C erhitzt. Nach dem Abkühlen wird das Reaktionsgemisch mit $CH_2Cl_2$ extrahiert, die organische Phase mit verdünnter HCl gewaschen und dann mit $Na_2SO_4$ getrocknet und eingeengt. Der Rückstand wird chromatographiert (Kieselgel, Hexan $\rightarrow$ $CH_2Cl_2$/Methanol) und das Produkt unter Zusatz von Aktivkohle aus Ethanol umkristallisiert. Ausbeute 0,91 g (63%).

b) 1-Chlor-4,10-ophenylenacridon

0,9 g (2,8 mmol) N-(2-Carboxyphenyl)-2-chlorcarbazol werden in 15 g Polyphosphorsäure 6 h bei 160 °C erhitzt und das Gemisch dann hydrolysien. Das Hydrolysat wird mit Toluol/Tetrahydrofuran (1:1) extrahiert, die organische Phase mit Wasser gewaschen, über $Na_2SO_4$ getrocknet und eingeengt. Der Rückstand wird chromatographiert (Kieselgel, $CH_2Cl_2$/Hexan (1:1) $\rightarrow$ $CH_2Cl_2$/Diethylether). Man erhält 175 mg (21%) 1-Chlor-4,10-o-phenylenacridon und 373 mg (44%) des Stellungsisomeren 12-Chlor-4,10-o-phenylenacridon.

c) 1-Phenoxy-4,10-o-phenylenacridon

175 mg (0,58 mmol) 1-Chlor-4,10-o-phenylenacridon werden zusammen mit 320 mg (3,4 mmol) Phenol und 560 mg (4 mmol) $K_2CO_3$ in 5 ml Dimethylsulfoxid 24 h bei 90 °C erhitzt. Nach dem Abkühlen giesst man die Reaktionsmischung in verdünnte wässrige NaOH (10%) und extrahiert mit Toluol/Tetrahydrofuran (2:1). Die organische Phase wird mit verdünnter wässriger NaOH (10%) und dann mit gesättigter Kochsalzlösung gewaschen, danach über $Na_2SO_4$ getrocknet und eingeengt. Der Rückstand wird chromatographiert (Kieselgel, $CH_2Cl_2$). Man erhält 20 mg (10%) der Titelverbindung, Schmelzpunkt 276-279 °C.

Beispiel A10: Herstellung von 5-(p-Carboxyphenoxy)imidazo[4,5,1-de]acridin-6-on.

a) 1(p-Carboxyphenoxy)-4-nitro-acridon

2g (7,28 mmol) 1-Chlor-4-nitro-acridon, 1g (7,28 mmol) 4-Hydroxybenzoesäure, 20 ml Dimethylformamid und 4,03 g (29,12 mmol) $K_2CO_3$ werden 18 h bei 60 °C und 6 h bei 80 °C gerührt. Nach dem Abkühlen wird das Reaktionsgemisch unter Rühren in 2N HCl gegossen. Der Niederschlag wird abfiltriert, mit Wasser gewaschen und im Vakuum getrocknet. Der Rückstand wird mit Essigsäureethylester ausgekocht. Man erhält 2,53 g (92%) Produkt, Schmelzpunkt >260 °C.

b) 5-(p-Carboxyphenoxy)imidazo[4,5,1-de]acridin-6-on

1 g 1-(p-Carboxyphenoxy)-4-nitro-acridon, 20 ml Ameisensäure, 0,45 g Eisenspäne und 1 ml Wasser werden 6 h bei 90 °C gerührt, nochmals 1 ml Wasser und 0,25 g Eisenspäne zugegeben und weitere 18 h bei 90 °C gerührt. Das Reaktionsgemisch wird in Wasser gegossen, und mit Toluol/Tetrahydrofuran extrahiert. Die organische Phase wird über $Na_2SO_4$ getrocknet und eingedampft. Der Rückstand wird in 10 ml Ameisensäure aufgenommen und 2,5 h am Rückfluss gerührt. Dann wird in Wasser gegossen, der Niederschlag abfiltriert, mit Wasser gewaschen und im Vakuum getrocknet. Der Rückstand wird mit Essigsäureethylester ausgekocht. Man erhält 0,51 g (54%) der Titelverbindung, Schmelzpunkt >260 °C.

Beispiel A11: Herstellung von 5-(m-Carboxyphenoxy)imidazo[4,5,1-de]acridin-6-on.

a) 1-(m-Carboxyphenoxy)-4-nitro-acridon

Es wird unter Verwendung von m-Hydroxybenzoesäure gemäss Beispiel A10a verfahren. Man erhält 1,54 g (56%) Produkt, Schmelzpunkt >260 °C.

b) 5-(m-Carboxyphenoxy)imidazo[4,5,1-de]acridin-6-on

Es wird unter Verwendung von 1-(m-Carboxyphenoxy)-4-nitro-acridon gemäss Beispiel A10b verfah-

ren. Man erhält 0,33 g (35%) Produkt, Schmelzpunkt >260 °C.

B) Anwendungsbeispiele

Beispiele B1 bis B11:

Die Verbindungen der Beispiele A1 bis A11 werden als Schicht auf einen Glasträger aufgebracht und mit Licht einer Wellenlänge von 300400 nm bestrahlt, wobei sich die Umlagerungsprodukte B bilden. In der nachfolgenden Tabelle sind die Absorptionsmaxima in nm [$\lambda_{max}$(A)] der Ausgangsverbindungen A und der entsprechenden Umlagerungsprodukte B [$\lambda_{max}$(B)] der irreversiblen photochromen Systeme angegeben:

| Beispiel Nr. | Verbindung Nr. | [$\lambda_{max}$(A)] | [$\lambda_{max}$(B)] |
|---|---|---|---|
| B1 | A1 | 353 | 440 |
| B2 | A2 | 354 | 530 |
| B3 | A3 | 357 | 542 |
| B4 | A4 | 356 | 529 |
| B5 | A5 | 357 | 535 |
| B6 | A6 | 358 | 548 |
| B7 | A7 | 359 | 517 |
| B8 | A8 | 359 | 532 |
| B9 | A9 | 302/395 | 597 |
| B10 | A10 | 357 | 534 |
| B11 | A11 | 456 | 534 |

**Patentansprüche**

1. Verbindungen der Formeln I,

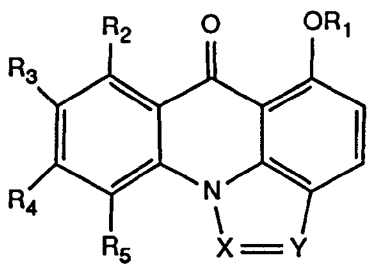

(I),

worin
X und Y je N oder $CR_6$ oder X $CR_6$ und Y N bedeuten;
$R_1$ unsubstituiertes oder mit $C_1$-$C_{12}$-Alkyl, $C_1$-$C_{12}$-Alkoxy, $C_1$-$C_{12}$-Alkylthio, $C_1$-$C_{12}$-Alkylsulfinyl, $C_1$-$C_{12}$-Alkylsulfonyl, Phenyl, Benzyl, Phenylsulfinyl, Benzylsulfinyl, Phenylsulfonyl, Benzylsulfonyl, -CN, $-CF_3$, Halogen, $-SO_3R_7$, $-CO_2R_7$, $-CO_2R_7$, $-CON(R_6)_2$, $-N(R_6)_2$ substituiertes $C_6$-$C_{14}$-Aryl darstellt, wobei das Phenyl und das Phenyl im Benzyl unsubstituiert oder mit $C_1$-$C_4$-Alkyl oder $C_1$-$C_4$-Alkoxy substituiert ist;
$R_2$, $R_3$, $R_4$ und $R_6$ unabhängig voneinander für H, $-CO_2R_7$ oder Halogen stehen;
$R_6$ H, unsubstituiertes oder mit $C_1$-$C_4$-Alkyl, $C_1$-$C_4$-Alkoxy, $C_1$-$C_4$-Alkylthio, $-CO_2R_7$ oder Halogen substituiertes $C_1$-$C_{18}$-Alkyl, $C_3$-$C_8$-Cycloalkyl, $C_6$-$C_{14}$-Aryl oder $C_7$-$C_{20}$-Aralkyl ist, oder beide $R_6$ zusammen die Gruppe -CH=CH-CH=CH- bedeuten, wenn X und Y je $CR_6$ sind, wobei diese Gruppe unsubstituiert oder

wie für $R_6$ definiert sbstituert ist;
$R_7$ für H, $C_1$-$C_{18}$-Alkyl, Cyclohexyl, Cyclopentyl, Phenyl, $C_1$-$C_{12}$-Alkylphenyl, Benzyl oder $C_1$-$C_{12}$-Alkylbenzyl steht; und
die $R_8$ unabhängig voneinander H, $C_1$-$C_{18}$-Alkyl, Cyclohexyl, Cyclopentyl, Phenyl, $C_1$-$C_{12}$-Alkylphenyl, Benzyl, $C_1$-$C_{12}$-Alkylbenzyl ist, oder ein $R_8$ H und das andere $R_8$ die Gruppe -CO-$R_9$ bedeutet, worin $R_9$ unbhängig die Bedeutung von $R_7$ hat, oder beide $R_8$ zusammen Tetramethylen, Pentamethylen, 3-Oxa-1,5-pentylen oder den Rest der Formel $-CH_2CH_2-N(C_1-C_6\text{-Alkyl})CH_2CH_2-$ darstellen.

2. Verbindungen gemäss Anspruch 1, dadurch gekennzeichnet, dass $R_2$, $R_3$, $R_4$ und $R_8$ für H stehen.

3. Verbindungen gemäss Anspruch 1, dadurch gekennzeichnet, dass $R_1$ unsubstituiertes oder substituiertes $C_6$-$C_{10}$-Aryl darstellt.

4. Verbindungen gemäss Anspruch 3, dadurch gekennzeichnet, dass $R_1$ unsubstituiertes oder substituiertes Phenyl darstellt.

5. Verbindungen gemäss Anspruch 1, dadurch gekennzeichnet, dass $R_1$ unsubstituiert oder mit $C_1$-$C_4$-Alkyl, $C_1$-$C_4$-Alkoxy, Phenyl, Benzyl, $-CF_3$, F, Cl, Br, $-SO_3R_7$ und $-CO_2R_7$ substituiert ist, wobei $R_7$ $C_1$-$C_4$-Alkyl bedeutet.

6. Verbindungen gemäss Anspruch 1, dadurch gekennzeichnet, dass $R_1$ unsubstituiertes Phenyl ist.

7. Verbindungen gemäss Anspruch 1, dadurch gekennzeichnet, dass $R_6$ als Alkyl $C_1$-$C_{12}$-Alkyl ist.

8. Verbindungen gemäss Anspruch 7, dadurch gekennzeichnet, dass $R_6$ $C_1$-$C_6$-Alkyl ist.

9. Verbindungen gemäss Anspruch 7, dadurch gekennzeichnet, dass das Alkyl mit F, Cl, $C_1$-$C_6$-Alkoxy und $-CO_2(C_1-C_4\text{-Alkyl})$ substituiert ist.

10. Verbindungen gemäss Anspruch 1, dadurch gekennzeichnet, dass R6 als Cycloalkyl Cyclopentyl oder Cyclohexyl ist.

11. Verbindungen gemäss Anspruch 10, dadurch gekennzeichnet, dass das Cycloalkyl mit F, Cl, $C_1$-$C_6$-Alkoxy und $-CO_2(C_1-C_4\text{-Alkyl})$ substituiert ist.

12. Verbindungen gemäss Anspruch 1, dadurch gekennzeichnet, dass $R_6$ als Aryl $C_6$-$C_{10}$ Aryl ist.

13. Verbindungen gemäss Anspruch 12, dadurch gekennzeichnet, dass das Aryl Phenyl ist.

14. Verbindungen gemäss Anspruch 1, dadurch gekennzeichnet, dass $R_6$ als Aralkyl $C_7$-$C_{12}$-Aralkyl ist.

15. Verbindungen gemäss Anspruch 14, dadurch gekennzeichnet, dass das Aralkyl Benzyl ist.

16. Verbindungen gemäss Anspruch 12 oder Anspruch 14, dadurch gekennzeichnet, dass das Aryl und das Aryl im Aralkyl mit $C_1$-$C_4$-Alkyl, $C_1$-$C_4$-Alkoxy, F, Cl und $-CO_2(C_1-C_4\text{-Alkyl})$ substituiert ist.

17. Verbindungen gemäss Anspruch 1, dadurch gekennzeichnet, dass $R_7$ als Alkyl $C_1$-$C_{12}$-Alkyl, als Alkylphenyl $C_1$-$C_4$-Alkylphenyl und als Alkylbenzyl $C_1$-$C_4$-Alkylbenzyl darstellt.

18. Verbindungen gemäss Anspruch 1, dadurch gekennzeichnet, dass $R_8$ als Alkyl $C_1$-$C_{12}$-Alkyl, als Alkylphenyl $C_1$-$C_4$-Alkylphenyl und als Alkylbenzyl $C_1$-$C_4$-Alkylbenzyl darstellt.

19. Verbindungen gemäss Anspruch 1, dadurch gekennzeichnet, dass $R_9$ lineares oder verzweigtes $C_1$-$C_6$-Alkyl, Phenyl oder Benzyl darstellt.

20. Verbindungen gemäss Anspruch 1, dadurch gekennzeichnet, dass X und Y je N oder $CR_6$ oder X $CR_8$ und Y N bedeuten; $R_1$ unsubstituiertes oder mit $C_1$-$C_6$-Alkyl, $C_1$-$C_6$-Alkoxy, -CN, $-CF_3$, F, Cl, Br, $-SO_3R_7$, $-CO_2R_7$ oder $-CON(R_8)_2$ substituiertes Phenyl darstellt; $R_2$, $R_3$, $R_4$ und $R_8$ unabhängig voneinander für H oder $-CO_2(C_1-C_4\text{-Alkyl})$ stehen; $R_6$ H, unsubstituiertes oder mit $C_1$-$C_4$-Alkoxy substituiertes $C_1$-$C_{12}$-Alkyl, unsubstituiertes oder mit $C_1$-$C_4$-Alkyl, $C_1$-$C_4$-Alkoxy, $-CO_2(C_1-C_4\text{-Alkyl})$, F, Cl oder Br substituiertes $C_5$- oder $C_6$-Cycloalkyl, Phenyl oder Benzyl ist, oder beide $R_6$ zusammen die Gruppe -CH=CH-CH=CH- bedeuten, wenn X und Y je $CR_6$ sind; $R_7$ H, $C_1$-$C_4$-Alkyl, Cyclohexyl, Phenyl oder Benzyl bedeutet und $R_6$ für $C_1$-$C_4$-Alkyl, Phenyl oder Benzyl steht.

**21.** Verfahren zur Herstellung von Verbindungen der Formel I, dadurch gekennzeichnet, dass man

a) zur Herstellung von Verbindungen mit X gleich $CR_6$ und Y gleich N

i) eine Verbindung der Formel II

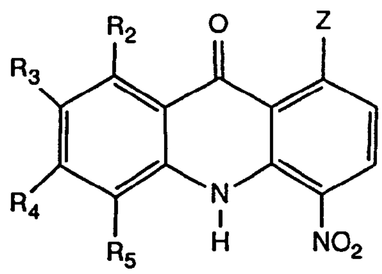

(II)

worin $R_2$, $R_3$, $R_4$ und $R_6$ die zuvor angegebenen Bedeutungen haben und Z für Cl oder Br steht, in Gegenwart eines polaren aprotischen oder protischen Lösungsmittels und bei erhöhter Temperatur mit einer Verbindung der Formel $R_1O^{\ominus}M^{\oplus}$ umsetzt, worin $R_1$ die zuvor angegebenen Bedeutungen hat und $M^{\oplus}$ für ein Alkalimetall- oder ein Ammoniumkation steht,

ii) die gebildete Verbindung der Formel III

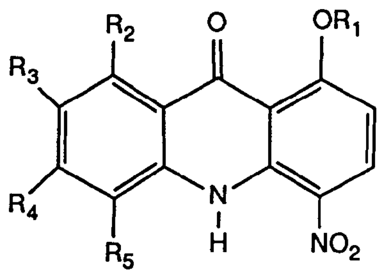

(III)

zu einer Verbindung der Formel IV reduziert

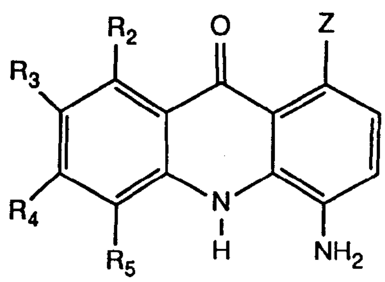

(IV),

und

iii) die Verbindung der Formel IV danach mit einer Carbonsäure der Formel $R_6$-$CO_2H$ umsetzt;

b) zur Herstellung von Verbindungen der Formel I, worin X und Y je N oder $CR_6$ bedeuten eine Verbindung der Formel V

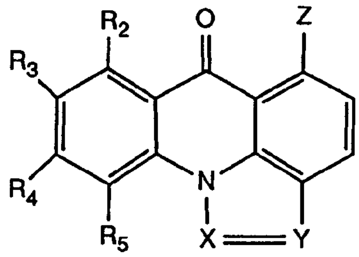

(V),

worin $R_2$, $R_3$, $R_4$, $R_5$ und Z die zuvor angegebenen Bedeutungen haben, in Gegenwart eines polaren aprotischen oder protischen Lösungsmittels und bei erhöhter Temperatur mit einer Verbindung der Formel $R_1O^{\ominus}M^{\oplus}$ umsetzt, worin $R_1$ die zuvor angegebenen Bedeutungen hat und $M^{\oplus}$ für ein Alkalimetall- oder ein Ammoniumkation steht.

**22.** Zusammensetzung, enthaltend
a) ein strahlungsempfindliches organisches Material, und
b) mindestens eine Verbindung der Formeln I gemäss Anspruch 1.

**23.** Zusammensetzung gemäss Anspruch 22, dadurch gekennzeichnet, dass die Verbindungen der Formel I in einer Menge von 0,001 bis 20 Gew.-% enthalten sind, bezogen auf die Komponente a).

**24.** Zusammensetzung gemäss Anspruch 22, dadurch gekennzeichnet, dass es sich bei dem strahlungempfindlichen organischen Material um al) eine nichtflüchtige monomere, oligomere oder polymere Substanz mit photopolymerisierbaren oder photodimerisierbaren ethylenisch ungesättigten Gruppen, a2) um ein kationisch härtbares System oder a3) um photovernetzbare Polyimide handelt.

**25.** Zusammensetzung, enthaltend
a) ein farbloses organisches Lösungsmittel, ein Polymer oder ein organisches Glas oder ein Verbundglas, und
b) gelöst, eingemischt oder als Schicht auf mindestens einer Oberfläche eine Verbindung der Formel I gemäss Anspruch 1.

**26.** Zusammensetzung gemäss Anspruch 25, dadurch gekennzeichnet, dass die Komponente b) in einer Menge von 0,001 bis 20 Gew.-%, enthalten ist, bezogen auf Komponente a).

**27.** Verfahren zur Herstellung von gefarbten Materialien unter Einwirkung von Licht, dadurch gekennzeichnet, dass man dem Material eine Verbindung der Formel I gemäss Anspruch 1 einverleibt und darauf das Material mit Licht bestrahlt.

**28.** Verwendung der Verbindungen der Formel I gemäss Anspruch 1 als Photosensibilisatoren und Farbindikatoren oder photoschaltbare Farbfilter bei Lichteinwirkung.

**29.** Verwendung der Verbindungen der Formel I gemäss Anspruch 1 als photochrome Systeme zur Kontrastbildung oder Lichtabsorption.